# EUROPEAN PATENT APPLICATION

(11) **EP 4 832 005 A1**
(43) Date of publication of application: **28.10.2026**
(21) Application number: 25171696.5
(22) Date of filing: 22.04.2025
(51) Int. Cl.: G16H 40/67, G01R 33/28, G01R 33/3815, H01F 6/02

(54) **SUPPORTING AN OPERATION OF A SUPERCONDUCTING MAGNET**

(71) Applicant: Koninklijke Philips N.V., 5656 AE Eindhoven (NL)
(72) Inventor: AMTHOR, Thomas Erik, Eindhoven (NL); FORTHMANN, Peter, Eindhoven (NL)
(74) Representative: Philips Intellectual Property & Standards

(57) **Abstract**

Disclosed is a computer-implemented method of supporting an operation of a superconducting magnet generating a magnetic field at a magnet site. The method comprises, by a control unit: receiving event information describing events related to the magnet site; monitoring the event information for fulfillment of a predefined emergency criterion that describes a situation potentially requiring a power-down of the magnet; and if the emergency criterion is fulfilled: using a remote assistance session that is established via a computer network between the control unit and a remote processing unit for instructing the remote processing unit to perform a power-down assistance setup routine that comprises outputting, via an assistant user interface of the remote processing unit, a notification about a potential need to power down the magnet; and enabling the remote processing unit to use an endpoint function of an endpoint device at the magnet site via the remote assistance session.

## Description

### FIELD OF THE INVENTION

The invention relates to the field of using a collaborational session conveyed via a computer network for operative support of a superconducting magnet.

### BACKGROUND OF THE INVENTION

The health care industry is facing unprecedented challenges including increasing patient volumes, increasing rates of burnout and turnover among healthcare professionals as well as increasingly strained resources. To help medical facilities and healthcare providers adapt to such changes, new technologies and solutions are being made available to enable increased workflow efficiency and productivity with fewer staff members. Since the advent of the internet, telecommunication and remote control solutions providing new possibilities of remote collaboration and assistance have become widespread not only in the medical field.

In the field of radiology, solutions are known that enable remote support for operators of medical imaging devices and remote image acquisition from a central assistance facility, allowing expert imaging technologists to support on-site health professionals and technologists at medical imaging sites anywhere in the world.

Magnetic resonance imaging (MRI) is an imaging technique that typically involves operating a superconducting magnet to generate a sufficiently strong magnetic field in a sufficiently large volume. A superconducting magnet is an electromagnet made from coils of superconducting wire. They must be cooled to cryogenic temperatures during operation. In its superconducting state the wire has no electrical resistance and therefore can conduct much larger electric currents than ordinary wire, creating intense magnetic fields. They are used not only in MRI instruments in hospitals, radiology facilities or other healthcare providers, but also in scientific equipment such as nuclear magnetic resonance (NMR) spectrometers, mass spectrometers, fusion reactors and particle accelerators.

In many applications, it is desirable to set a superconducting magnet into persistent mode where a continuous electric current through the superconducting wire generates a static magnetic field as long as the magnet is cryogenically kept in its superconducting state. The magnetic field may be powered up by executing a so-called ramp-up procedure that slowly increases the electric current until the desired magnetic field strength is reached. Analogously, a superconducting magnet may be powered down without losing superconductivity by applying a slow ramp-down procedure.

When a superconducting magnet generating a magnetic field loses its superconductivity, the ohmic resistance of the wire suddenly assumes a non-zero value. As a consequence, the electric energy stored in the magnetic field and the electric current through the magnet's windings is converted to heat in a short time. This event is called a magnet quench and may cause a rapid boil-off of the cryogenic coolants, which is usually liquid helium for low-temperature superconducting (LTS) magnets, or other coolants as required for keeping the respective superconductor material of the magnet below the critical temperature. An unplanned quench of a large superconducting magnet should be avoided as it may result in damages and / or potentially fatal injury due to the sudden release of large amounts of electrical and / or thermal energy and coolant vapor.

A magnet quench may cause a significant downtime. It can take several days for the magnet to be in operation again after such an event. For these reasons, a magnet quench can typically only be initiated by personnel on site through a hardware button. Personnel may be specially trained and authorized to initiate a magnet power-down only when there is a clear indication for it.

EP 3 407 081 A1 describes an MRI system with an emergency button that can be actuated in a first way to initiate a procedure of ramping down a superconducting magnet, and in a second way to initiate a procedure of quenching the superconducting magnet.

### SUMMARY OF THE INVENTION

It is an objective to provide for an improved computer-implemented method of supporting an operation of a superconducting magnet generating a magnetic field at a magnet site, and a correspondingly configured computer program product and control unit as well. The objectives underlying the invention are solved by the features of the independent claims.

In one aspect, there is disclosed a computer-implemented method of supporting an operation of a superconducting magnet that generates a magnetic field at a magnet site. The method comprises, by a control unit, receiving event information that describes events related to the magnet site. The method further comprises, by the control unit, monitoring the event information for fulfillment of a predefined emergency criterion. The emergency criterion describes a situation that potentially requires a power-down of the magnet. The method further comprises, by the control unit, if the emergency criterion is fulfilled, instructing a remote processing unit to perform a power-down assistance setup routine. The remote processing unit is instructed using a remote assistance session that is established via a computer network between the control unit and the remote processing unit. The power-down assistance setup routine comprises outputting, via an assistant user interface, a notification about a potential need to power down the magnet. The method further comprises, by the control unit, if the emergency criterion is fulfilled, enabling the remote processing unit to use an endpoint function of an endpoint device at the magnet site via the remote assistance session.

In another aspect, there is disclosed a non-transitory computer-readable medium. The medium stores instructions that, when executed by one or more processors, cause the one or more processors to perform at least the following steps. The steps comprise receiving event information that describes events related to a magnet site. The steps further comprise monitoring the event information for fulfillment of a predefined emergency criterion. The emergency criterion describes a situation that potentially requires a power-down of a superconducting magnet generating a magnetic field at the magnet site. The steps further comprise, if the emergency criterion is fulfilled, instructing a remote processing unit to perform a power-down assistance setup routine. The remote processing unit is instructed using a remote assistance session that is established via a computer network between the control unit and the remote processing unit. The power-down assistance setup routine comprises outputting, via an assistant user interface, a notification about a potential need to power down the magnet. The steps further comprise, if the emergency criterion is fulfilled, enabling the remote processing unit to use an endpoint function of an endpoint device at the magnet site via the remote assistance session.

In a further aspect, there is disclosed a system. The system comprises a processor and a memory that is coupled to the processor. The system is configured to receive event information that describes events related to a magnet site. The system is further configured to monitor the event information for fulfillment of a predefined emergency criterion. The emergency criterion describes a situation that potentially requires a power-down of a superconducting magnet generating a magnetic field at the magnet site. The system is further configured to instruct a remote processing unit to perform a power-down assistance setup routine if the emergency criterion is fulfilled. The remote processing unit is instructed using a remote assistance session that is established via a computer network between the control unit and the remote processing unit. The power-down assistance setup routine comprises outputting, via an assistant user interface, a notification about a potential need to power down the magnet. The system is further configured to enable the remote processing unit to use an endpoint function of an endpoint device at the magnet site via the remote assistance session if the emergency criterion is fulfilled.

In a further aspect, a system comprising a processor and a memory coupled to the processor is described. The system is configured to receive, obtain or otherwise acquire, event information describing adverse events related to an MRI device of a plurality of MRI devices; monitor the event information for fulfillment of a predefined emergency criterion based on calculated probability of an adverse event occurring and describing a situation potentially requiring a power-down of the magnet. If the emergency criterion is fulfilled, using a remote assistance session, established via a computer network, to remotely perform a power-down assistance setup routine. At least in some embodiments, the power-down assistance setup routine is providing for one or more of: sequence of steps a user of the MRI device of the plurality of MRI devices needs to perform to power-down the magnet, establishing a remote control by a remote user of the MRI device of the plurality of MRI device, or establishing an emergency communication session between the remote user and the user of the MRI device.

These and other aspects of the invention will be apparent from and elucidated with reference to the example(s) described hereinafter. It is understood that one or more of the aforementioned examples may be combined as long as the combined examples are not mutually exclusive.

### BRIEF DESCRIPTION OF THE DRAWINGS

In the following, examples are described in greater detail making reference to the drawings in which:
Fig. 1 illustrates an environment where a method of supporting an operation of a superconducting magnet may be executed;
Fig. 2 is a flow chart illustrating steps of a method of supporting an operation of a superconducting magnet;
Fig. 3 is a block diagram showing components of a control unit; and
Fig. 4 is a block diagram showing components of an endpoint device.

### DETAILED DESCRIPTION OF EMBODIMENTS

As described below in greater detail, examples may unfold beneficial effects by facilitating a power-down of a superconducting magnet in situations that could have prevented or impeded a shutdown of the magnet previously. Moreover, examples may provide for enhanced operational safety of the superconducting magnet by allowing for a remotely conducted or assisted prospective power-down of the magnet in apparently or actually impending emergency situations.

Although magnetic resonance imaging (MRI) is used herein as a primary application scenario of a superconducting magnet for demonstrating traits of the invention, it is understood that methods, steps, ideas, approaches, principles and other elements described herein may likewise be applied to other usage scenarios involving a deployment of a superconducting magnet.

When, e.g., in an MRI setting, a magnetic-field-induced accident happens, a second knowledgeable person is typically required to initiate an emergency quench of the magnet. This may not always be the case, for instance if a radiology department is understaffed, or during evening or early morning hours. Furthermore, there may be situations when adverse external events cause a safety risk that would require a prospective power-down of the magnet. While these events may be known shortly in advance, there may not always be local staff available to safely shut down the magnetic resonance (MR) system.

The method of supporting an operation of a superconducting magnet generating a magnetic field at a magnet site described herein may be executed by a control unit. The control unit may be understood as a computing device (e.g., a server, a workstation, a gateway, a mainframe, etc.) that is equipped at least with a processor, a memory functionally coupled to the processor, and a communication interface. The memory of the control unit may store instructions that are executable by the processor and which, when executed by the processor, cause the control unit to implement the method.

The control unit may use the communication interface to exchange information with the remote processing unit and one or more endpoint device(s) (in the following abbreviated as "the endpoints") located at the magnet site. For this purpose, the communication interface may comprise a network interface that allows for exchanging information with the remote processing unit, and optionally also with the endpoints, via the computer network (e.g., the internet, a local area network (LAN), a wireless (Wi-Fi) network, etc., and combinations thereof). Alternatively, communication between the control unit and the endpoints may be realized by local communication infrastructure of the magnet site such as a bus, a local-area network (LAN), a private network, etc. that is separate from the computer network. In this scenario, the communication interface may further comprise an endpoint interface in addition to the network interface. From a network point of view, the control unit may be disposed in an interfacing position between the endpoints and the remote control unit.

The remote processing unit may be regarded as a server, a personal computer or a terminal computer having integrated and / or connected thereto typical user interfaces such as input devices and output devices, and having a network interface allowing an exchange of data between the remote processing unit and the computer network.

The superconducting magnet (herein also referred to as "the magnet") is located at a magnet site. The magnet site may be considered as a building, floor, department or other facility that is equipped for operating the superconducting magnet. Besides the superconducting magnet, the magnet site may host one or more endpoint device(s). An endpoint device may be an electric or electronic device having an endpoint function and a signal interface in communicative connection with the endpoint function. The communicative connection between the signal interface and the endpoint function may be realized in many ways, including without limitation electrical, electromagnetic / optical, electromechanical, and / or electrochemical connections.

The endpoint function may be any function of the endpoint device that is configured for generating information (e.g., a sensor function), and / or for facilitating a user interaction (in particular, an actuation, an input interaction and / or an output interaction) between a person at the magnet site and the endpoint device, and for exchanging information (e.g., data and / or a control signals) with the signal interface. Thus, the signal interface may facilitate an exchange of information, including transfer of data and / or control signals, between the endpoint function and another device, such as the control unit, that is in communicative connection to the signal interface. The following non-exclusive examples illustrate possible implementations of an endpoint device.

In an example, the endpoint device is an indicator device that comprises an indicator unit as the endpoint function and a wired or wireless communication interface as the signal interface. Without limitation, an indicator unit may be a display screen, a lamp, a loudspeaker, a computer, or another device configured for or capable of generating output that is perceivable for persons at the magnet site. Receipt of a corresponding signal via the signal interface may trigger the indicator unit to generate an output that is independent of the received signal, or may cause the indicator unit to generate an output that is specified by the received signal. For instance, the received signal may cause: the indicator unit being a lamp or LED to emit light that is visible at the magnet site; the indicator unit being a display screen to display a static image, a video and / or text information to be seen at the magnet site; the indicator unit being a loudspeaker to generate a sound that is audible at the magnet site; the indicator device being a static or mobile computer to generate respective output by operating a hardware function, executing a software function, starting a media playback; etc. The received signal may influence the output or may control the endpoint function. For instance, the received signal may: set the color and / or intensity of the light emitted by the lamp or LED; comprise image, video, text, audio and / or media data to be output by the display screen, loudspeaker or computer; comprise a control signal and / or a computer-executable command specifying one or more actions of controlling the hardware and / or software function; etc.

In another example, the endpoint device is a switch that comprises a button as the endpoint function and the signal interface comprising a wired or wireless communication interface operatively coupled to an auxiliary switch operating unit. In a first mode of operation, a person at the magnet site may operate the switch by manually actuating the button. In a second mode of operation, a signal received via the signal interface may cause the auxiliary switching unit to operate the switch without said manual actuation of the button. In order to use the second mode of operation, the received signal may optionally have to match an expected signal for unlocking the second mode of operation. The auxiliary switching unit may be configured for operating the switch in many ways, including without limitation a circuitry for short-circuiting or bypassing the button, or an electromechanical unit such as a robot arm or other actuator to actuate the button without a manual user action.

The method includes the step of receiving event information that describes events related to the magnet site. This may include various kinds of information originating from the magnet site or pertaining to a geographical location of the magnet site. Events related to the magnet site may include events potentially or actually occurring at the magnet site. Event information may be any information that actually or potentially includes information that allows for determining, using the predefined emergency criterion described herein, whether a situation potentially requiring a power-down of the magnet is currently happening or likely to occur. The following examples are intended to give a non-exhaustive, non-limiting illustration of the concept of the event information.

In an example, a portion of the event information is generated by a sensor measuring a quantity that describes a property of the magnet site. In an example, the sensor is located at the magnet site. In an example, the sensor located at the magnet site is a camera, a photo sensor, or a microphone. In an example, the sensor is an emergency sensor configured for detecting an indication of an adverse event, a risk event, or an emergency event. In an example, the emergency sensor is a heat sensor, a smoke sensor, a gas sensor (e.g., adapted for detecting carbon dioxide (CO2), carbon monoxide (CO), or chlorine (Cl2)), an accelerometer, a temperature sensor, a wind speed sensor, or a water sensor. In an example, the emergency sensor is located at the magnet site. In an example, the sensor is configured for monitoring the superconducting magnet or a physical property related to the magnet. In an example, the physical property related to the magnet is a temperature of the magnet, a temperature of a thermal bath of the magnet, a strength of the magnetic field, a pressure inside a housing of the magnet, or a temperature inside a room comprising the magnet. A sensor monitoring the magnet may be a camera overseeing a room where the magnet is installed, or a microphone picking up sound in the magnet room. Sensors providing event information may include sensors that are part of an alarm infrastructure installed at the magnet site.

In an example, a portion of the event information is generated by a user interface at the magnet site. In an example, the user interface at the magnet site generating the portion of the event information is a camera, a microphone, an emergency switch, or an input device of a computer located at the magnet site.

In an example, a portion of the event information is generated by an information service. In an example, the information service is a weather report service, a weather forecast service, a news service, or an emergency alert service. In an example, the emergency alert service is adapted for generating as the portion of the event information an alert describing an actual or potential occurrence of: a fire, an earthquake, a volcanic activity, a tsunami, a flooding, a bomb threat, an extreme weather event, or an evacuation request. An information service may be a public service, which does not necessarily mean that the information service is free of charge, but rather that it is publicly accessible using, e.g., the computer network if it is a public network.

In an example, a portion of the event information is generated by a risk event monitoring device. Apart from sensors located at the magnet site and suiting the purpose of detecting an indication of a risk event, the risk event monitoring device may be located elsewhere and may be configured for detecting an indication of a risk event that potentially or actually affects the magnet site. In an example, the risk event monitoring device is a seismometer, a weather station, a weather satellite, an air quality sensor, or a water level gauge.

The control unit may monitor these and other examples of event information to determine whether it is indicative of a situation that potentially requires a power-down of the magnet (e.g., by ramping down the magnetic field, quenching the magnet, or performing another routine allowing for eliminating the magnetic field generated by the superconducting magnet and / or electric currents generating said magnetic field). For this purpose, the control unit may have available a predefined emergency criterion. The monitoring may include reading the event information and comparing the read event information to numerical and / or logical terms (e.g., keywords, threshold values, etc.) that the emergency criterion contains to define which situations that are reflected by the event information may be situations that may require a power-down of the magnet. In some examples, an event can refer to an adverse event, i.e., that there is a potential adverse event (e.g., earthquake) happening at the magnet site.

The control unit, or another computing device (e.g., a node of a cloud computing infrastructure that the control unit can contact via the computer network) instructed accordingly by the control unit, may conduct a deeper analysis of the event information. Analysis of the event information by the control unit may be preferable if privacy of the event information may be protected better this way. The analysis may improve comparability of the event information to the emergency criterion and to facilitate an identification of such situations that are not included verbatim in the event information. Without limitation, techniques of analyzing the event information may include speech recognition to identify keywords or other hints expressed in spoken language that may indicate an emergency situation (e.g., a cry for help); sound feature recognition to identify audible events that may indicate a potential emergency situation (e.g., a bumping sound); image feature or object recognition to identify features visible in image or video data that may indicate an emergency situation (e.g., an object such as a wheelchair or a bed covering the bore of the magnet, a person being pressed to a housing of the magnet, a hurricane approaching the magnet site, etc.); sound pattern detection to identify features audible in audio data that may indicate an emergency situation ; and / or numerical data analysis to identify signatures of a potential emergency situation in numerical data (e.g., seismographic data showing signs or precursors of an earthquake). Analysis of the event information may be performed using numerical algorithms and / or artificial intelligence (AI) models, including but not limited to machine learning (ML) models that are trained for the respective analysis task.

If the emergency criterion is fulfilled, the control unit may contact a remote processing unit via the computer network. This may be done by establishing a remote assistance session via the computer network or using an existing remote assistance session between the control unit and the remote processing unit. The remote assistance session may be regarded as a connection through the computer network that allows for exchange of information between the control unit and the remote processing unit, which optionally includes telecommunication in at least one direction and / or a capability of remotely controlling one or more devices at the magnet site. The remote assistance session may require authentication at least for an operator (also referred to herein as "the assistant") of a user interface ("the assistant user interface") of the remote processing unit or connected thereto (e.g., using a wired or wireless connection). In an application scenario, the control unit is a workstation adapted for controlling an MRI system comprising the superconducting magnet. In this scenario, the remote assistance session may require authentication for an operator of the control unit (e.g., a medical or technical user of the MRI system) and the assistant as well, and may provide, for instance, capabilities for bidirectional audiovisual real-time communication and remote control of the MRI system by the assistant using the assistant user interface.

The remote processing unit may be located at a place (also referred to as "the assistance center") where a distance between the assistant and the magnet site exists that is sufficiently large that a power-down of the magnet cannot realistically be performed by manual intervention of the assistant at the magnet site. In an example, the magnet site is a large building or complex of buildings and both the control unit and the remote processing unit are located at the magnet site, but are separated from each other so far that, in an emergency situation, it would take unacceptably long for the assistant to leave the assistant user interface and go to the magnet to power down the magnet themselves. In another example, the assistance center is located at a location that is geographically separate from the magnet site (e.g., in another city or country) and offers the remote assistance session as a service to the magnet site, and optionally, to other customers operating a superconducting magnet as well. One example of such service may be the Radiology Operations Command Center (ROCC) from Philips.

Using the remote assistance session, the control unit may instruct the remote processing unit to perform a power-down assistance setup routine by transmitting a corresponding power-down assistance setup instruction to the remote processing unit. The power-down assistance setup routine may comprise a step of outputting a notification ("the setup notification") via the assistant user interface that informs the assistant that there is a potential need to power down the magnet. For this purpose, the power-down assistance setup instruction may comprise an identifier of the magnet, a system (e.g., an MRI system) containing the magnet, and / or the magnet site to cause the remote processing unit to include a corresponding identification information in the output and thus enable the assistant to correctly identify the magnet or its corresponding system or magnet site. The power down assistant setup routine may comprise further steps such as those described herein or other steps that may facilitate preparing the assistant for a potential power-down of the magnet. The setup notification may be provided to the assistant as a visual and / or audible output of the assistant user interface.

The setup notification may enable the assistant to become aware of the potential emergency situation at the magnet site and to take steps of getting an own understanding of the situation. For this purpose, the power-down assistance setup routine may execute further steps of preparing the assistant user interface with functions (also referred to as "assistance functions") and / or additional information ("the setup information") that the assistant may use to provide assistance, via the remote assistance session, that may enable a person located at the magnet site to power down the magnet; to prepare the assistance; and / or to decide whether the magnet should actually be powered down or not. Without limitation, the assistance functions may include communication functions as well as support for selecting, contacting, and / or remotely controlling an endpoint. Without limitation, the setup information may include current information about the magnet, a potential system hosting the magnet, the magnet site, devices and / or persons located at the magnet site, and / or portions of the event information that contributed to the fulfilment of the emergency criterion, optionally accompanied by portions of the event information that are related to said contributing portions of the event information. The remote processing unit may receive portions of the setup information from the control unit and / or from other sources such as a database ("the site database") that may be available, for instance, via the computer network.

Furthermore, the method comprises, if the emergency criterion is fulfilled, enabling the remote processing unit to use an endpoint function of an endpoint device at the magnet site via the remote assistance session. As described below in greater detail, the endpoint may be selected by the assistant using the assistant user interface, or automatically by the control unit or the remote processing unit. The enablement of using the endpoint function of an endpoint at the magnet site may further enhance the assistant's capabilities of preparing or providing assistance in powering down the magnet. The following non-limiting selection of application scenarios illustrates examples of the assistant's capabilities provided by the control unit, the remote assistance session, and the remote processing unit.

In an example, the assistant may use the remote assistance session to verify if there is evidence justifying a power-down of the magnet. For this purpose, the assistant may access additional information sources via the assistant user interface. For instance, if the setup notification indicates that the fulfilment of the emergency criterion indicates a potential extreme weather event at the magnet site, the assistant may retrieve a detailed weather report or an image generated by a meteorological satellite to assess a weather situation related to the magnet site.

In another example, the setup notification indicates that the fulfilment of the emergency criterion indicates a potential accident at the magnet. The assistant may verify this by signaling to the control unit that they would like to connect to a camera (the endpoint device) monitoring a room of the magnet site where the magnet is installed. The control unit may respond thereto by enabling the remote processing unit to use an endpoint function of the endpoint device (in this case, the video capturing function of the camera). If, e.g., the camera supports additional endpoint functions of tilting and zooming, it may also be possible that the control unit enables the remote processing unit to use these functions, too. This could enable the assistant to remotely control the tilt and zoom functions of the camera via the remote assistance session to gather more details for understanding whether the alleged accident has really occurred, and if so, if a remedy for the accident requires an emergency quench of the magnet.

In another example, the control unit selects an endpoint at the magnet site that provides real-time audiovisual communication as an endpoint function (e.g., an intercom device, a tablet computer, etc.) and has a high probability of enabling a successful power-down of the magnet. For instance, the endpoint may be selected by determining which accordingly equipped endpoint is closest to the magnet, has been used for telecommunication most recently, is located near a comparably large number of persons at the magnet site, and / or other suitable criteria for determining said success probability. The control unit may then enable the remote processing unit to use the endpoint function of the selected endpoint device via the remote assistance session by establishing a telecommunication connection between the selected endpoint and the assistant user interface. The assistant may use this connection to call for a person at the magnet site (e.g., by the control unit causing the endpoint to output a signal indicating an incoming call, or by causing the endpoint to display a video stream of the assistant being captured by a camera and a microphone connected to the remote processing unit).

In another example, the remote assistance session provides for a real-time communication connection between the assistant and a person at the magnet site ("the responding person" or "the operator") when or after the assistant receives the setup notification. This may be the case, for instance, when a person answers the call above, or when the communication connection has already been established before the fulfilment of the emergency criterion was detected. The assistant, having verified that there is indeed a need to power down the magnet, may then use the communication connection to guide or instruct the responding person towards performing the power-down of the magnet. In order to do so, the assistant may use the remote assistance session to interact with the assistant user interface, the remote processing unit, and the control unit to use one or more endpoint(s) accordingly. In response, the control unit may perform one or more actions of enabling the remote processing unit to use an endpoint function of an endpoint device at the magnet site via the remote assistance session. For instance, the assistant may communicate with the operator at the magnet site to explain the steps that the operator should carry out to power down the magnet; remotely control indicator devices (e.g., lamps, actuators, displays, loudspeakers, ...) to visualize actions the operator should perform, or locations the operator should go to, to prepare or carry out the power-down of the magnet, or other devices such as remotely controllable door locks to enable the operator to power down the magnet; and / or switch the real-time communication connection from one endpoint to another (e.g., by manually selecting the next endpoint via the assistant user interface, or by automatic tracking of the operator's location by the control unit based on the event information) to accompany the operator as they change location carrying out the assistant's instructions.

In another example, the assistant may use the remote assistance session to power down the magnet themselves, without interaction by a responding person at the magnet site. In this scenario, the control unit may enable the remote processing unit to use a power-down hardware switch (the endpoint device) to trigger (the endpoint function) an automatic power-down routine (e.g., ramping down the magnetic field, or quenching the magnet) controlled by a power-down module of the magnet. This may be practicable or feasible if the magnet is an HTS magnet requiring a reduced cryogenic setup compared to an LTS magnet, and / or if no person at the magnet site is responding the assistant's call. Possible implementations of remotely controlling a switch such as the power-down hardware switch are described herein. In the example of a superconducting magnet being part of an MRI system, the power-down hardware switch may be realized, e.g., as a hardware button connected to a heating system inside the magnet to initiate a quench; or as a hardware button connected to a power unit of the magnet to ramp down the magnetic field in a controlled fashion.

A magnet power-down remotely controlled by the assistant may be facilitated by the assistance functions comprising a "remote power down" element displayed on the assistant user interface. In an example, the control unit may enable the remote processing unit to use the trigger function of the power-down switch in response to receiving a signal from the remote processing unit that indicates that the assistant has activated the remote power down element. In an example, the remote power down element is blocked on startup of the power-down assistance setup routine and an additional remote power down element unblocking criterion has to be fulfilled in order to enable the assistant to activate the remote power down element by unblocking the remote power down element. For instance, the remote power down element unblocking criterion may include the assistant providing additional credentials to prove that they are authorized to perform a remotely controlled magnet power-down; the remote processing unit receiving from the endpoint a confirmation signal indicating that an operator at the magnet site agrees to the remote power-down; and / or a predefined unblock delay time has passed to ensure that the remote power down element is not activated accidentally or without sufficient consideration.

Examples of the method described herein may have the advantage of facilitating a power-down of a superconducting magnet in situations that could have prevented or impeded a shutdown of the magnet previously. As it is common good practice to have an expert perform or witness a power-down routine for a superconducting magnet, thanks to the remote assistance session the expert does not have to be physically present at the magnet or the magnet site if the method is applied. The may be especially beneficial at daytimes when the magnet site is staffed with a reduced number of personnel; for understaffed departments where persons knowledgeable of superconducting magnets are present at the magnet site with a reduced number of hours per week; for autonomous imaging booths that offer an MRI system hosting a superconducting magnet; and / or in emergency situations when personnel at the magnet site is busy with emergency response or evacuation.

Moreover, examples of the method may provide for enhanced operational safety of the superconducting magnet by allowing for a remotely conducted or assisted prospective power-down of the magnet in apparently or actually impending emergency situations. A prospective power-down may increase safety by preventing an uncontrolled magnet quench that would set free large amounts of energy and / or coolant vapor in a short time.

Multiple additional advantages may become apparent when considering details of the method. For example, connecting the assistant to communication endpoints at the magnet site via the remote assistance session may ensure an improved availability compared to, e.g., mobile networks that may be overloaded or unavailable during an emergency situation of public scope. For example, the automatic monitoring of the event information (that may be received continuously) may allow for generating the setup notification at an early time when the assistant may be unaware that there is a situation potentially requiring a power-down of the magnet based on information channels other than the remote assistance session. For example, the control unit may be considered as a gateway to the endpoints that may be configurable to provide security functions preventing unauthorized access to the endpoints, e.g., by unauthorized persons potentially using the assistant user interface or using a computing device apart from the remote processing unit for circumventing the remote assistance session. The gateway function of the control unit may also enable to lower technical requirements for the remote processing unit, e.g., as the control unit may have available some or all drivers, application programming interfaces (APIs), etc. that are necessary for operating the endpoints at the magnet site with their various endpoint functions.

In an example, the monitoring further comprises determining an emergency probability score based on the event information, the emergency criterion being fulfilled if the emergency probability score equals or exceeds a predefined emergency probability threshold, the instructing of the remote processing unit to perform the power-down assistance setup routine comprising transmitting the emergency probability score to the remote processing unit, the notification about a potential need to power down the magnet being indicative of the emergency probability score. This may support the assistant in deciding whether there is an actual need to power down the magnet, and to obtain a first idea of how urgent the situation at the magnet site appears. For instance, a fulfilment of the emergency criterion based on an emergency indication triggered by an interaction of a person at the magnet site with one of the endpoints (e.g., an emergency button was pressed) may be automatically assigned a high value (e.g., 95% or 100%) as it can be assumed that a person has already assessed the situation at the magnet site and concluded that there is an emergency situation ongoing or impending. In another example, the emergency probability score may be an inverse measure of the uncertainty of an image recognition algorithm in detecting a predefined emergency indication in the event information as part of the monitoring. In another example, the emergency probability score is obtained by reading the event information if, for instance, the event information already comprises the emergency probability score, for example in a weather warning indicating a probability that an extreme weather event may occur at a geographical location of the magnet site. The emergency probability score, and optionally the emergency probability threshold, may be presented to the assistant via the assistant user interface, using, e.g., a percentage or other number representation, a diagram, colors of a predefined color palette, or a combination thereof.

In an example, the instructing of the remote processing unit to perform the power-down assistance setup routine comprises transmitting to the remote processing unit an event specification descriptive of the situation, the notification about a potential need to power down the magnet being descriptive of the situation based on the event specification. This may support the assistant in deciding whether there is an actual need to power down the magnet, and to obtain a first idea of a potential impact the situation at the magnet site may have on the operation of the magnet. Based on the event specification, the setup notification (i.e., the notification about a potential need to power down the magnet) may inform the assistant about the ongoing or potential emergency situation using a corresponding text (e.g., predefined or included in the power-down assistance setup instruction transmitted by the control unit to the remote processing unit), an audible speech output, a color code assigned to a predefined category of the event specification, etc.

In an example, the instructing of the remote processing unit to perform the power-down assistance setup routine comprises transmitting to the remote processing unit a site specification describing the magnet site, the site specification enabling the power-down assistance setup routine to display a representation of the endpoint device within a site plan of the magnet site via the assistant user interface, the representation being based on a current location of the endpoint device. In this manner, the method may enhance the assistant's capabilities of interacting with persons and / or devices at the magnet site. The site specification may enable the remote processing unit to display a representation of the site plan to the assistant via the assistant user interface. This may enable the assistant to plan and coordinate potential steps toward a remotely assisted power-down of the magnet via the remote assistance session. The site plan may contain a map of a building, floor or other building part, department, ... where the magnet is located. In the following, the site plan and any other information obtained using the site specification (including, without limitation, current information about available endpoints) are together referred to as "the site information".

In an example, the site specification enables the remote processing unit to retrieve the site information from a site database that is available via the computer network. In an example, the control unit retrieves the site information from the site database and transmits the retrieved information to the remote processing unit as part of the site specification. In an example, the site plan comprises a current layout of rooms at the magnet site, optionally with room numbers and representations of infrastructure such as doors, stairs, elevators, supply lines, the position of the magnet, and / or current positions of the endpoints. In an example, the representation of the site plan provides the assistant with a function of selecting one or more of the displayed endpoints for connecting via the remote assistance session and the control unit. In an example, the representation of the site plan provides the assistant with a function of displaying, in response to the assistant selecting one of the displayed endpoints, additional information about the endpoint, such as a device type and / or a list of available endpoint functions of the selected endpoint. The representation of the site plan may include various further functions enhancing the assistant's interactivity capabilities, such as colors or pictograms visualizing, e.g., the type of some or all endpoints; a function of highlighting a selected endpoint and / or an endpoint for which the usage by the remote processing unit was successfully enabled; functions of filtering endpoints based on their device type and / or supported endpoint functions (e.g., real-time communication capability, indicator functions, loudspeakers, video playback support, hardware buttons, remotely controllable endpoints, sensors (optionally allowing for subcategory filtering such as smoke detectors, cameras, microphones, etc.); and many more.

The site information may also facilitate automatic endpoint selection by the control unit and / or the remote processing unit. The site database may also store personnel information such as assignments of names, phone numbers, departments, ... that may be displayed to the assistant as well. This may enable the assistant to select a particular communication endpoint based on characteristics of persons who are located near the particular endpoint. Additional security measures may be implemented for the site database as it may store sensitive information such as assignments of names, phone numbers, departments, ... and infrastructure information that should not be disclosed to the public such as floor plans, etc.

In an example, the enabling of the remote processing unit to use the endpoint function further comprises:
in response to the instructing of the remote processing unit to perform the power-down assistance setup routine, receiving from the remote processing unit a communication request indicating an intention to start a real-time communication session;
in response to the receipt of the communication request, instructing the endpoint device to output via the endpoint function a call message asking for using the endpoint device to reply to the communication request, the call message being perceivable at the magnet site.

As explained before, this may enable the assistant to search for an operator who can communicate with the assistant and can power down the magnet at the magnet site according to the assistant's instructions. The call message may, for instance, indicate that the endpoint device is receiving an emergency response call. If the endpoint outputting the call message has a display, it may be instructed to output, e.g., a representation of the assistant (e.g., a photograph of the assistant's face), an identifier of a manufacturer of the magnet or a system (e.g., an MRI system) hosting the magnet, and / or other information that may help a potential operator to understand the purpose of the incoming call and to decide whether to answer the call or not. Communication endpoints may include all endpoints that allow for real-time communication between a person at the magnet site and the remote assistant. Possible communication endpoints may include, without limitation: the tablet or touch screen device mounted anywhere at the magnet site, including user interface devices connected to the control unit and / or user interfaces in non-restricted areas of the magnet site; personal mobile devices such as mobile phones carried by staff of the magnet site; communication software installed on a desktop personal computer (PC), for example at a reception desk; etc.

In an example, the method further comprises, in response to the instructing of the endpoint device to output the call message:
receiving a reply signal indicating a usage of the real-time communication capability by the endpoint device;
in response to the receipt of the reply signal, transmitting via the computer network to an emergency response system a magnet operator notification indicating that a person is staying at the magnet site to perform the quench of the magnet.

This may indicate, e.g., to emergency response units that the person stays at the magnet site instead of leaving the building, as may be expected in some emergency situations. The magnet operator notification does not necessarily have to identify the person staying at the magnet site.

In an example, the enabling of the remote processing unit to use the endpoint function further comprises obtaining an indication of a designated endpoint device, and enabling the remote processing unit to use, via the remote assistance session, an endpoint function of the designated endpoint device as the endpoint function of the endpoint device, the obtaining of the indication of the designated endpoint device comprising:
determining the indication of the designated endpoint device by selecting the designated endpoint device from a plurality of endpoint devices present at the magnet site, the selected endpoint device having, among the plurality of endpoint devices, a high probability of allowing for successfully performing the power-down of the magnet using the remote assistance session, and instructing the remote processing unit to output a representation of the selected endpoint device via the assistant user interface; or
receiving the indication of the designated endpoint device from the remote processing unit.

This may facilitate determining for which of the available endpoints the control unit should perform the enabling of the remote processing unit to use the respective endpoint function. If the control unit selects the selected endpoint device, it may, for instance, analyze the site information described herein to determine the probability of allowing for successfully performing the power-down of the magnet using the remote assistance session ("the success probability") for some or all of the available endpoints. This may be done in various ways, such as based on determining a number of persons located in a predefined vicinity around a given endpoint (e.g., all persons within a radius or a walking distance of 25 meters around the given endpoint), wherein an endpoint neighboring many persons may be assigned a higher success probability than an endpoint neighboring a small number of persons; a potential qualification of persons near a given endpoint to power down the magnet, where, e.g., a technical qualification may be assigned a higher success probability than an unknown qualification of a visitor or a patient; available endpoint functions of the respective endpoints, where, e.g., endpoint functions matching a requested endpoint function (e.g., as explicitly input by the assistant using an endpoint filter function, or as prescribed by the currently activated endpoint sequence) may be assigned a higher success probability than mismatching endpoint functions; a distance of an endpoint from the magnet or from a location of the magnet site where the potential emergency situation is occurring, where, e.g. endpoints may be assigned a higher success probability with decreasing distance from the magnet and / or increasing distance from the potential emergency situation); etc. In an example, the control unit follows a predefined sequence of endpoints to be used, e.g., to guide a person toward a power-down switch of the magnet. In an example, the sequence of endpoints may be determined in response to obtaining an indication of an initial endpoint and determining which endpoints of the plurality of endpoints are closest to a shortest path connecting the initial endpoint to the power-down switch. In an application scenario, the qualification of persons near an endpoint may be obtained by analyzing records of staff who has registered with a presence capture system using their digital ID card when entering, e.g., the department where the magnet is located.

Alternatively or additionally, the control unit may receive the indication of the designated endpoint device from the remote processing unit. The remote processing unit, in turn, may obtain the indication of the designated endpoint device as an input of the assistant via the assistant user interface, or by executing an endpoint selection routine similar to the selection of the endpoint by the control unit outlined above.

In an example, the method further comprises receiving presence information indicating current locations of persons at the magnet site, the selection of the designated endpoint device being based on the presence information. This may increase the likelihood that a person is going to answer the assistant's call to be output using a given endpoint. The presence information may be received, e.g., as part of the site information. For instance, an endpoint may be selected according to the number of persons near this endpoint, and / or according to the distance of persons from this endpoint. As explained before, the presence information may also allow for tracking the responding person after an initial endpoint has been selected. As the responding moves away from a previous endpoint and approaches a next endpoint, the control unit may switch off the enabling of the remote processing unit to use an endpoint function of the previous endpoint and switch on an enabling of the remote processing unit to use an endpoint function of the next endpoint. In this manner, the assistant may accompany the responding person using varying endpoints without substantial interruptions of communication.

In an example, the enabling of the remote processing unit to use the endpoint function further comprises enabling a remote control of the endpoint function by the remote processing unit. This may enhance the assistant's capabilities of assisting the responding person in preparing or conducting the power-down of the magnet. For instance, the assistant may control endpoints that are light sources or actuators to show the operator how to operate functions of a technical system that is unknown to the operator, or to guide the operator along a path through the premises of the magnet site. Enabling remote control capability may include communicating available endpoint functions of the remotely controllable endpoint to the remote processing unit for providing an indication of the available endpoint functions to the assistant via the assistant user interface; and / or instructing the remote control unit to output via the assistant user interface one or control element(s) allowing for remotely controlling the available endpoint function(s).

In an example, the endpoint device is an indicator device configurable, by receipt of an activation information, for providing an indication for performing a manual action associated with enabling the power-down of the magnet, the indication for performing a manual action associated with enabling the power-down of the magnet being perceivable at the magnet site, the method further comprising transmitting the activation information to the endpoint device. As indicated above, this may enable a responding person to follow instructions of the assistant even if the operator is untrained. A remotely controllable indicator system may be used to guide local untrained users performing a safety-critical operations instructed by the remote assistant. For instance, indicators may be switched on and off by the assistant and may be individual hardware devices that can be mounted on walls, attached to doors, or next to equipment needed for preparing or conducting the power-down of the magnet. Indicators may include, but are not limited to: lamps or flashing lights; mechanically rotating or vibrating devices; screens adapted for displaying information; etc. For instance, indicator lights or actuators may be attached to required buttons (door openers, quench button, etc.) that the responding person needs to recognize when following the assistant's instructions.

Possible scenarios of transmitting the activation information to the endpoint device may include transmitting the activation information as specified by a script that may be executed by the control unit or the remote processing unit and may implement a particular sequence or conditions defining when to activate the indicator device; and / or in response to an input of the assistant to the remote processing unit indicating the assistant's intention to activate the indicator device. In examples, the activation information may comprise a switch signal configured for switching the indicator device on or off, and / or a data payload such as video data and / or audio data to be output by the indicator device.

In an example, the method further comprises, in response to receiving from the remote processing unit an information indicating an intention to activate the indicator device, providing the activation information to the indicator device according to a predefined time pattern specific to the indicator device. This may facilitate the responding person to follow the assistant's instructions. For instance, the time pattern may cause the control unit to switching on the endpoint after a delay time specific to the endpoint, which may, e.g., give the operator sufficient time to execute a preceding step; switching on and off the endpoint in a time sequence specific to the endpoint, which may, e.g., help the operator to discern visually similar endpoints. In an example, the indicator device is a display and the time pattern causes a delayed video playback via the display to increase the probability that the operator is able to pay attention to the video. In an example, the endpoint is a light source and the time pattern causes the light source to flash at a specific repetition rate to help the operator find a particular element in a confusing environment.

In an example, the endpoint function is configured to trigger the power-down of the magnet; or to trigger a buildup of the magnetic field. This may enable the assistant to perform a remotely controlled power-down of the magnet themselves and / or to remotely power up the magnet again after ensuring that the emergency situation is over. Powering a superconducting magnet up or down using remote control may be feasible or practicable for HTS magnets and may require specially configured ramp-down or quenching equipment.

In an MRI application scenario, a local interface device (e.g., a relay) may be deployed for connecting at least the affected MRI device(s) with a magnet power-down unit. To start the ramp-down, a local processing unit may activate a local interface device based on a request of the remote assistant, which may in turn interact with the MRI system hardware by either mechanically pressing a quench button using a robotic button pusher or by short-circuiting a switch for triggering the quench or ramp-down procedure. In this way, remotely controllable power-down capability may be retrofittable to MRI systems of multiple vendors. In a variant, the magnet power-down functionality is available via software, which may allow for replacing the relay / actuator by a network connection to the control unit, an MRI imaging console, or other MRI host computer.

Alternatively or additionally, the assistant user interface may be instructed to output an element providing a function to ramp up the magnet. This function may require an HTS-type magnet. Ramping up a magnet remotely may be beneficial in some situations that are demonstrated by the following MRI application scenarios. For instance, a remote procedure of building up the magnetic field may be performed if the magnet had been turned off over night and needs to be turned on again for the first patient in the morning, but local staff is late; if a magnet that was intended not to be used (e.g., during the night) is needed on short notice for an emergency patient. With an HTS magnet, it may be possible that the assistant can remotely handle the ramp-up procedure while on-site staff is preparing for patient arrival. For remotely powering up a superconducting magnet, an enhanced setup including room cameras may be beneficial so that the assistant can ensure that there is no person in the room during the field buildup procedure to limit safety risks.

In an example, the method further comprises obtaining a verification whether an assistant operating the assistant user interface is authorized for using the endpoint function, the enabling of the remote processing unit to use the endpoint function being performed if the obtaining of the verification is successful. This may increase security of the method by ensuring that no unauthorized person can use the assistant user interface to power down the magnet or use other endpoint functions available at the magnet site in an unauthorized manner. The verification may be obtained by receiving the verification from the remote processing unit after successfully verifying the assistant's identity and / or privileges; or by analyzing credential information received from the remote processing unit as to whether it fulfils a predefined authorization criterion. If the verification is obtained successfully, the control unit may enable the remote processing unit to use the endpoint function. Optionally, upon finishing the enablement, the control unit may instruct the remote processing unit to unblock a control element that is output via the assistant user interface and is adapted for triggering the use of the endpoint function (e.g., the remotely controlled power-down of the magnet); or the remote processing unit may unblock the control element upon successful verification of the assistant's privileges.

In an example, the method further comprises, in response to receiving, from the remote processing unit, a trigger information indicating an intention to trigger the power-down of the magnet or an intention to trigger the buildup of the magnetic field:
instructing a notification endpoint device at the magnet site to output a trigger notification, the trigger notification being perceivable at the magnet site and providing a function for manually preventing the triggering indicated by the trigger information within a predefined trigger delay time using the notification endpoint device;
in response to receiving within the predefined trigger delay time from the notification endpoint device a prevention information indicating the manual prevention: preventing the triggering indicated by the trigger signal; and instructing the remote processing unit to output, via the assistant user interface, a prevention notification indicating that the triggering was canceled by a user at the magnet site.

This may increase the safety and security of the method by enabling an operator at the magnet site to stop an attempt of powering the magnet up or down within the trigger delay time. If the operator does not react or no operator is present (for example, if the operator is involved in the emergency situation), the power-down may be performed after the trigger delay time. For this purpose, the control unit may cause an endpoint having the requested endpoint function of triggering the magnet power-down or the magnetic field buildup to execute the requested endpoint function. In response to receiving the prevention information from the notification endpoint device, the control unit may disable the remote processing unit to use the triggering endpoint function that was requested by the trigger information. In response to receiving from the notification endpoint device an agreement signal indicating a user's agreement to triggering the power-down of the magnet or the buildup of the magnetic field, respectively, the control unit may continue by causing the endpoint having the requested triggering endpoint function to perform the requested triggering immediately, with no regard to a possibly remaining portion of the trigger delay time. A reasonable range for the trigger delay time may be from 5 to 600 seconds, preferably from 10 to 180 seconds. For the case that an operator is near (within an operating range of) the notification endpoint, a reasonable range for the trigger delay time may be from 10 to 20 seconds. In response to receiving the prevention information from the notification endpoint device, the control unit may instruct the remote processing unit to output via the assistant user interface a prevention notification indicating to the assistant that the attempt to power down the magnet or build up the magnetic field has been manually canceled by a user at the magnet site. In response to not receiving the prevention signal from the notification endpoint device within the predefined trigger delay time, the triggering indicated by the trigger signal may be executed, e.g., by the control unit instructing the endpoint device to execute the triggering, or by the endpoint device performing the triggering autonomously in response to detecting that the trigger delay time has passed.

In an application example, the assistant may have been unsuccessful in trying to establish a real-time communication connection to an endpoint because nobody at the magnet site responded to the call signal. As a next step, the assistant decided to trigger a remote power-down of the magnet, thus starting a trigger delay time that, in this example, is preset to 3 minutes. During the trigger delay time, a person at the magnet site who is unaware of the call signal notices the attempt of remotely powering down the magnet by reading the trigger notification on a display of the notification endpoint device (e.g., a portable computer or mobile phone) and activates a control element displayed by the notification endpoint device to prevent the triggering of the magnet power-down by transmitting the prevention information to the control unit. The control unit may respond thereto by stopping the countdown of the trigger delay time, disabling the conditional performance of the requested power-down, and notifying the assistant with a (e.g., predefined) prevention notification.

In an example, the enabling of the remote processing unit to use the endpoint function further comprises unlocking an external interface of the endpoint device to enable a data transfer between the endpoint device and the remote processing system via the external interface. This may provide additional security against unauthorized use of the endpoint function via the external interface. The external interface may be an interface of the individual endpoint (such as the signal interface described above), or an external interface of the control unit or another control device controlling access to some or all of the endpoint devices by devices located outside the magnet site or an internal network of the magnet site. The control unit may unlock the external interface, e.g., by authenticating the usage of the external interface by the control unit using a cryptographic key; by providing to the endpoint or the respective control device a cryptographic certificate confirming the assistant's authorization to interact with endpoints at the magnet site; or by setting a parameter of the endpoint, the control unit, or the respective control device regulating the access to the endpoint(s) by external devices.

In an example, the event information is received from: a sensor at the magnet site; a user interface at the magnet site; an information service; a risk event monitoring device; or a combination thereof. These sources of the event information may allow for a comprehensive determination of potential or actual emergency situations potentially requiring a power-down of the superconducting magnet for a wide range of emergency types. Possible sources of the event information have been discussed above, together with possible kinds of emergency situations that may be detectable using event information provided by these sources.

In the following, reference is made to the drawings, where similar elements are denoted by the same reference numerals.

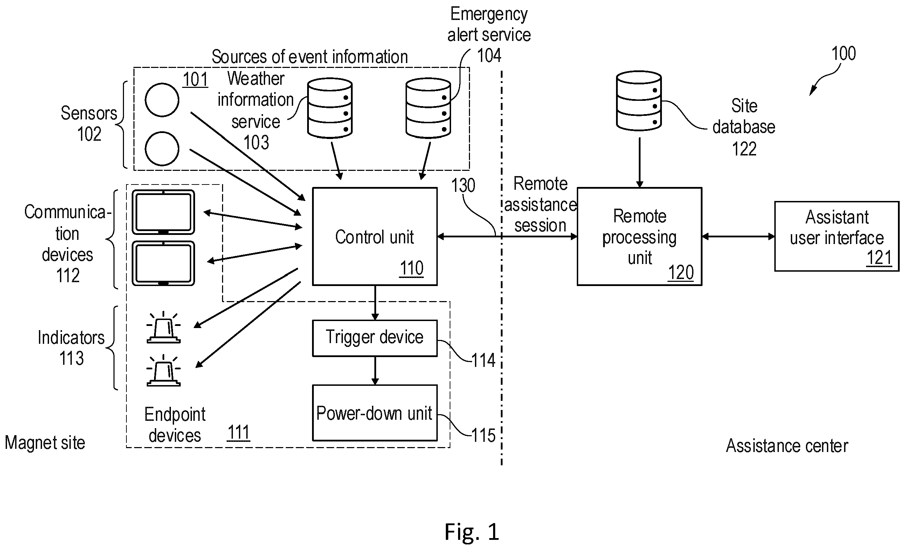

Fig. 1 shows an exemplary environment 100 that may be suitable for executing implementations of the method 200. According to the example of Fig. 1, the environment 100 may comprise a control unit 110 (e.g., a computing device) that is communicatively coupled to a remote processing unit 120 (e.g., another computing device) via a computer network. The computer network may allow for data transfer between devices located at a magnet site and devices located at an assistance center. In the example of Fig. 1, the control unit 110 is located at the magnet site and the remote processing unit 120 is located at the assistance center. The control unit 110 and the remote processing unit 120 may be configured to set up a remote assistance session 130 via the computer network. The remote assistance session 130 may allow for transferring information between the control unit 110 and the remote processing unit 120, including, but not limited to, a video stream, an audio stream, device control signals, graphical user interface output, and other data or signals.

The control unit 110 may receive event information from sources 101 of event information. In the example of Fig. 1, the sources 101 of event information include sensors 102 (e.g., emergency detection sensors and / or site monitoring sensors such as cameras and microphones installed at the magnet site), a weather information service 103 (e.g., a weather forecast service and / or a weather report service), and an emergency alert service 104 (e.g., a public risk event alert service). The sources 101 of event information may provide the event information to the local processing unit 110 via the computer network and / or other communication infrastructure. The weather information service 103 and / or the emergency alert service 104 may be examples of information services described herein.

The control unit 110 may exchange signals (e.g., signals for controlling an endpoint function of an endpoint device 111, and / or signals indicating user interactions with an endpoint device 111) and other information (e.g., data to be output by an endpoint device 111, and / or data that is input to an endpoint device 111) with endpoint devices 111 located at the magnet site. In the example of Fig. 1, the endpoints 111 may comprise communication devices 112 that may include, without limitation, real-time communication devices (e.g., display devices, loudspeakers, static or mobile computers, and / or intercom devices), indicator devices 113 (e.g., lamps and / or actuatable signaling devices), and a magnet power-down unit 115 having a mechanical user interface (e.g., a button) and a trigger device 114 (e.g., a relay or an actuator) that is controllable by the control unit 110 to trigger a function of the power-down unit 115 (e.g., a routine of powering down the magnet) by actuating the mechanical user interface of the power-down unit 115.

The remote processing unit 120 may be connected to an assistant user interface 121. The assistant user interface 121 may comprise user interface elements that may be integrated with the remote processing unit 120 and / or attached to the remote processing unit 120. Exemplary elements of the assistant user interface 120 include a computer keyboard, a computer mouse, a camera (e.g., a webcam), a microphone, a loudspeaker, etc. The remote processing unit 120 may receive setup information, as defined herein, from a site database 122 that may be hosted at the assistance center, at the magnet site, or by a node of the computer network. The control unit 110 and the remote processing unit 120 may also exchange information with other nodes of the computer network not shown in the drawing.

The environment 100 shown in the drawing has a particular configuration that has been chosen for the sole purpose of illustrating traits and notions of the invention. In this configuration, multiple elements of the environment 100 are shown with arrows symbolizing connections enabling data exchange and control flow. It is understood that numerous other configurations of the environment 100 may exist which may be suitable as well for executing implementations of the method 200. In particular, elements or connections shown in the drawing as components of the environment 100 may be missing in such alternative configurations; elements and connections not shown in the drawing may be components of the environment 100 in such alternative configurations; and connections shown in the drawing may have a different topology in such alternative configurations. More particularly, there may be more, less, further, or other sources 101 of event information than the sources 101 of event information shown in Fig. 1. There may be more, less, further, or other endpoint devices 111 than the endpoint devices 111 shown in Fig. 1. Units and elements shown in Fig. 1 such as the relay / actuator 114 and / or the power-down unit 115 may be missing in other examples. Some steps of the method 200, or portions thereof, may be executed by the remote processing unit 120 instead of the control unit 110 executing all steps of the method 200.

Similarly, the topology of connections between the elements of the environment 100 may differ from that shown in Fig. 1. For instance, the event information provided by some sources 101 of event information (e.g., the sensors 102) may be monitored and optionally analyzed by the control unit 110, while the event information provided by other sources 101 of event information (e.g., the weather information service 103 and / or the emergency alert service 104) may be monitored and optionally analyzed by the remote processing unit 120. In other words, it may be possible that steps or sub-steps of the method 200 can be performed by the remote processing unit 120 and optional further devices within the environment 100 in a distributed execution.

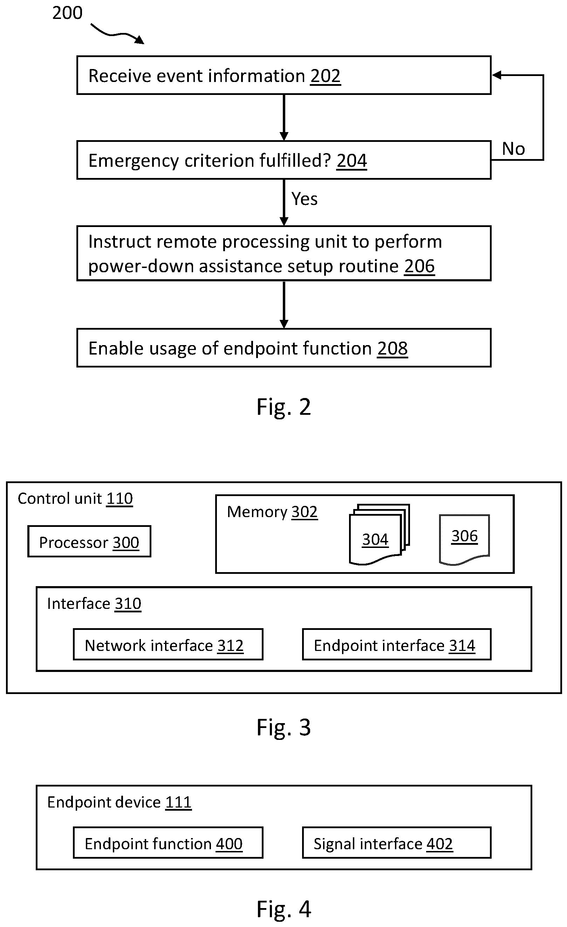

Fig. 2 is a flow chart illustrating steps of the method 200 of supporting an operation of a superconducting magnet generating a magnetic field at a magnet site. Although distributed execution may be possible, as explained above, the steps shown in Fig. 2 are described here from the point of view of a single control unit 110 performing all steps of the method 200, for the sake of simplicity of illustration.

The method 200 may comprise a step of receiving 202 event information. For instance, the control unit 110 may receive the event information from sources 101 of event information such as the sensors 102, the weather information service 103, and / or the emergency alert service 104. The event information may include portions of information describing events that may require a power-down of the superconducting magnet. The event information may be received continuously, e.g., as data that is generated continuously by surveillance and monitoring activities; and / or intermittently, e.g., in form of reports issued on a regular basis, and / or irregularly, e.g., on a case-by-case basis whenever an event of interest in a particular event category occurs.

In order to determine whether the received event information contains a portion of information describing a potential or actual event that may require a power-down of the superconducting magnet, the control unit 110 may monitor 204 the event information. The monitoring 204 may include comparing the event information, or a result of an analysis of the event information, to a predefined emergency criterion. The emergency criterion may describe one or more characteristics of data that may be present in the event information if the event information is indeed descriptive of a situation (e.g., an actual or potential emergency situation) that may require a power-down of the magnet. Optionally, analysis of portions of the event information may by performed by another device such as a node of a cloud computing infrastructure.

If the emergency criterion is not fulfilled, the control unit may continue with receiving 202 further event information and monitoring 204 the further event information. If the emergency criterion is fulfilled, the control unit 110 may use the remote assistance session 130 to the remote processing unit 120 that is established via the computer network. The remote assistance session 130 may be established if the monitoring 204 results in a detection of a fulfilment of the emergency criterion, or at an earlier time such that the control unit 110 may use the existing remote assistance session 130 to contact the remote processing unit 120.

Using the remote assistance session 130, the control unit 120 may instruct 206 the remote processing unit 120 to execute a power-down assistance setup routine. The power-down assistance setup routine may prepare the assistant user interface 121 in a manner that enables an assistant using the assistant user interface to contact a person at the magnet site via the remote processing unit 120, the remote assistance session 130, the control unit 110, and one or more of the endpoints 111. The preparation of the assistant user interface 121 may comprise retrieving information about the magnet site (e.g., a site plan of the magnet site, and / or positions of one or more of the endpoints 111) from a suitable information source such as the site database 122.

The control unit 110 may contribute to said enablement of the assistant by enabling 208 the remote processing unit 120 to use an endpoint function of an endpoint device 111 via the remote assistance session 130. This may require determining (e.g., by receiving a selection of an endpoint device 111 from the assistant via the assistant user interface 121; and / or by automatic selection of one or more of the endpoint devices 111 by a selection routine executed by the control unit 110 and / or the remote processing unit 120) at least one of the endpoints 111 for which the usage of an endpoint function by the remote processing unit 120 shall be enabled 208.

The assistant may then proceed with determining if a power-down of the magnet is really necessary or should rather be avoided. The information provided to the assistant via the assistant user interface 121 as a result of the power-down assistance setup routine, and / or a real-time communication to a responding person at the magnet site, may support the assistant in this decision. If the assistant can verify that there is indeed a need to power down the magnet, the assistant may use the remote assistance session 130 to, for example, contact a person at the magnet site by setting up a real-time communication session via one of the communication devices 112; and / or guide the responding person or operator at the magnet site by remotely controlling one or more of the indicators 113; and / or initiate the power-down of the magnet on their own behalf by using the trigger device 114 to trigger the power-down unit 115 of the system comprising the magnet.

Fig. 3 is a block diagram showing possible components of an exemplary control unit 110. The control unit 110 may comprise a processor 300, a memory 302, and an interface 310. The memory 302 and the interface 310 may be functionally coupled to the processor 310 such that information may be exchanged between the process of 300, the memory 302, and the interface 310. The memory 302 may store computer-executable programs and other compilations 304 of computer-executable code that may be executed by the processor 300. Among the programs and compilations 304 is a set of computer-executable instructions 306 which, when executed by the processor 300, caused the control unit 110 to carry out an implementation of the method 200. Interface 310 may be regarded as an external interface of the control unit 110 that may allow for an exchange of information between the control unit 110 and external devices. The interface 310 may comprise a network interface 312 that may enable an exchange of information between the control unit 110 and the computer network. In an example, the control unit 110 may exchange information with the remote processing unit 120 and one or more of the endpoint devices 111 via the computer network using the network interface 312. Optionally, the interface 310 may also comprise an endpoint interface 314 that may allow for exchanging information between the control unit 110 and one or more of the endpoint devices 111 via a separate network interconnecting the endpoint devices 111 and the control unit 110. Apart from the endpoint interface 314, the remote processing unit 120 may comprise components that are similar to the components of the control unit 110, including without limitation a processor similar to processor 300, a memory similar to the memory 302, and a network interface similar to the network interface 312.

Fig. 4 is a block diagram showing possible components of an exemplary endpoint device 111. The endpoint device 111 may comprise an endpoint function 400 and a signal interface 402. The endpoint function 400 may be implemented by various kinds of hardware units that may be part of endpoint device 111. For instance, the endpoint function 400 may be implemented by an output device (e.g., a lamp, a display, a loudspeaker, etc.) or an input device (e.g., a manually operatable switch having a button, a rocker, or other means allowing for manual operation of the switch; a computer keyboard, a computer mouse, a camera, a microphone, etc.). The endpoint function 400 may be implemented by an electrical, electronic, or electromechanical unit of the endpoint device 111 and may exchange data and / or other signals with the signal interface 402. For instance, the endpoint device 111 may receive via the signal interface 402 data to be output using the endpoint function 400 and / or a control signal adapted for controlling the endpoint function 400; and / or may transmit via the signal interface 402 data and / or signals that the endpoint device 111 received as an input to the endpoint function 400 and / or that are derived from such data and / or signals.

The signal interface 402 may be an external interface of the endpoint device 111 that may enable an exchange of information between the endpoint device 111 and external devices. For instance, the signal interface 402 may comprise an electrical or optical terminal, a network interface similar to the network interface 312 of the control unit 110, and / or another data exchange or signal exchange interface similar to the endpoint interface 314 of the control unit 110. Using the connections illustrated by Fig. 1, an exchange of signals (including data and / or control signals) may be established between the assistant user interface 121 the remote processing unit 120 using its network interface, the remote assistance session 130 established via the computer network, the control unit 110 using the network interface 312, and the endpoint device 111 using the interface 310 of the control unit 110, the signal interface 402 of the endpoint device 111, and the respective signal exchange infrastructure connecting the control unit 110 and the endpoint device 111.

While the invention has been illustrated and described in detail in the drawings and foregoing description, such illustration and description are to be considered illustrative or exemplary and not restrictive; the invention is not limited to the disclosed examples. It is understood that one or more of the aforementioned examples or embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer-executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer-executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer-executable code embodied therein, for example, in baseband or as part of a carrier wave. Such a propagated signal may take any of a variety of forms, including, but not limited to, electromagnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a computational system. 'Computer storage' or 'storage' is a further example of a computer-readable storage medium. Computer storage is any non-volatile computer-readable storage medium. In some embodiments computer storage may also be computer memory or vice versa.

A 'computational system' as used herein encompasses an electronic component which is able to execute a program or machine executable instruction or computer-executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer-executable code may comprise instructions or a program which causes a processor or other computational system to perform an aspect of the present invention. Computer-executable code for carrying out operations for aspects of the present invention may be written in any combination of one or more programming languages, including an object-oriented programming language such as Java, Smalltalk, C++ or the like and conventional procedural programming languages, such as the "C" programming language or similar programming languages and compiled into machine executable instructions. In some instances, the computer-executable code may be in the form of a high-level language or in a pre-compiled form and be used in conjunction with an interpreter which generates the machine executable instructions on the fly. In other instances, the machine executable instructions or computer-executable code may be in the form of programming for programmable logic gate arrays.

The computer-executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer-executable code when applicable. It is further understood that, when not mutually exclusive, combinations of blocks in different flowcharts, illustrations, and/or block diagrams may be combined. These computer program instructions may be provided to a computational system of a general-purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and / or tactile data. Examples of a display include, but are not limited to: a computer monitor, a television screen, a touch screen, tactile electronic display, Braille screen, Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### LIST OF REFERENCE NUMERALS

- 100: Environment
- 101: Sources of event information
- 102: Sensors
- 103: Weather information service
- 104: Emergency alert service
- 110: Control unit
- 111: Endpoint devices
- 112: Communication devices
- 113: Indicator devices
- 114: Trigger device
- 115: Power-down unit
- 120: Remote processing unit
- 121: Assistant user interface
- 122: Site database
- 130: Remote assistance session
- 200: Method
- 202: Receive event information
- 204: Monitor the event information
- 206: Instruct remote processing unit to perform power-down assistance setup routine
- 208: Enable usage of endpoint function
- 300: Processor
- 302: Memory
- 304: Programs / compilations of computer-executable code
- 306: Computer-executable instructions
- 310: Interface
- 312: Network interface
- 314: Endpoint interface
- 400: Endpoint function
- 402: Signal interface

## Claims

1. A computer-implemented method (200) of supporting an operation of a superconducting magnet generating a magnetic field at a magnet site, the method comprising, by a control unit (110):
receiving (202) event information describing events related to the magnet site;
monitoring (204) the event information for fulfillment of a predefined emergency criterion describing a situation potentially requiring a power-down of the magnet;
if the emergency criterion is fulfilled:
using a remote assistance session (130), established via a computer network between the control unit and a remote processing unit (120), for instructing (206) the remote processing unit to perform a power-down assistance setup routine, the power-down assistance setup routine comprising outputting, via an assistant user interface (121), a notification about a potential need to power down the magnet;
enabling (208) the remote processing unit to use an endpoint function (400) of an endpoint device (111) at the magnet site via the remote assistance session (130).

2. The method of claim 1, wherein the method further comprises:
the monitoring further comprises determining an emergency probability score based on the event information, the emergency criterion being fulfilled if the emergency probability score equals or exceeds a predefined emergency probability threshold, the instructing of the remote processing unit to perform the power-down assistance setup routine comprising transmitting the emergency probability score to the remote processing unit, the notification about a potential need to power down the magnet being indicative of the emergency probability score; or
the instructing of the remote processing unit to perform the power-down assistance setup routine comprises transmitting to the remote processing unit an event specification descriptive of the situation, the notification about a potential need to power down the magnet being descriptive of the situation based on the event specification; or
the instructing of the remote processing unit to perform the power-down assistance setup routine comprises transmitting to the remote processing unit a site specification describing the magnet site, the site specification enabling the power-down assistance setup routine to display a representation of the endpoint device within a site plan of the magnet site via the assistant user interface, the representation being based on a current location of the endpoint device; or
a combination thereof.

3. The method of any of the preceding claims, the enabling of the remote processing unit to use the endpoint function further comprising:
in response to the instructing of the remote processing unit to perform the power-down assistance setup routine, receiving from the remote processing unit a communication request indicating an intention to start a real-time communication session;
in response to the receipt of the communication request, instructing the endpoint device to output via the endpoint function a call message asking for using the endpoint device to reply to the communication request, the call message being perceivable at the magnet site.

4. The method of any of the preceding claims, the enabling of the remote processing unit to use the endpoint function further comprising obtaining an indication of a designated endpoint device, and enabling the remote processing unit to use, via the remote assistance session, an endpoint function of the designated endpoint device as the endpoint function of the endpoint device, the obtaining of the indication of the designated endpoint device comprising:
determining the indication of the designated endpoint device by selecting the designated endpoint device from a plurality of endpoint devices present at the magnet site, the selected endpoint device having, among the plurality of endpoint devices, a high probability of allowing for successfully performing the power-down of the magnet using the remote assistance session, and instructing the remote processing unit to output a representation of the selected endpoint device via the assistant user interface; or
receiving the indication of the designated endpoint device from the remote processing unit.

5. The method of claim 4, further comprising receiving presence information indicating current locations of persons at the magnet site, the selection of the designated endpoint device being based on the presence information.

6. The method of any of the preceding claims, the enabling of the remote processing unit to use the endpoint function further comprising enabling a remote control of the endpoint function by the remote processing unit.

7. The method of claim 6, the endpoint device being an indicator device (113) configurable, by receipt of an activation information, for providing an indication for performing a manual action associated with enabling the power-down of the magnet, the indication for performing a manual action associated with enabling the power-down of the magnet being perceivable at the magnet site, the method further comprising transmitting the activation information to the endpoint device.

8. The method of claim 7, further comprising, in response to receiving from the remote processing unit an information indicating an intention to activate the indicator device, providing the activation information to the indicator device according to a predefined time pattern specific to the indicator device.

9. The method of claim 6, the endpoint function being configured to trigger the power-down of the magnet; or to trigger a buildup of the magnetic field.

10. The method of claim 9, further comprising obtaining a verification whether an assistant operating the assistant user interface is authorized for using the endpoint function, the enabling of the remote processing unit to use the endpoint function being performed if the obtaining of the verification is successful.

11. The method of any one of claims 9 or 10, further comprising, in response to receiving, from the remote processing unit, a trigger information indicating an intention to trigger the power-down of the magnet or an intention to trigger the buildup of the magnetic field:
instructing a notification endpoint device at the magnet site to output a trigger notification, the trigger notification being perceivable at the magnet site and providing a function for manually preventing the triggering indicated by the trigger information within a predefined trigger delay time using the notification endpoint device;
in response to receiving within the predefined trigger delay time from the notification endpoint device a prevention information indicating the manual prevention: preventing the triggering indicated by the trigger signal; and instructing the remote processing unit to output, via the assistant user interface, a prevention notification indicating that the triggering was canceled by a user at the magnet site.

12. The method of any one of the preceding claims, the enabling of the remote processing unit to use the endpoint function further comprising unlocking an external interface of the endpoint device to enable a data transfer between the endpoint device and the remote processing system via the external interface.

13. The method of any one of the preceding claims, the event information being received from: a sensor (102) at the magnet site; a user interface at the magnet site; an information service (103, 104); a risk event monitoring device; or a combination thereof.

14. A non-transitory computer-readable medium storing instructions that, when executed by one or more processors, cause the one or more processors to:
receive (202) event information describing events related to a magnet site;
monitor (204) the event information for fulfillment of a predefined emergency criterion describing a situation potentially requiring a power-down of a superconducting magnet generating a magnetic field at the magnet site;
if the emergency criterion is fulfilled:
use a remote assistance session (130), established via a computer network between the control unit and a remote processing unit (120), for instructing (206) the remote processing unit to perform a power-down assistance setup routine, the power-down assistance setup routine comprising outputting, via an assistant user interface (121), a notification about a potential need to power down the magnet;
enable (208) the remote processing unit to use an endpoint function (400) of an endpoint device (111) at the magnet site via the remote assistance session.

15. A system comprising a processor (300) and a memory (302) coupled to the processor, the system configured to:
obtain (202) event information describing adverse events related to an MRI device of a plurality of MRI devices;
monitor (204) the event information for fulfillment of a predefined emergency criterion based on calculated probability of an adverse event occurring and describing a situation potentially requiring a power-down of the magnet, wherein if the emergency criterion is fulfilled,
using a remote assistance session (130), established via a computer network, to remotely perform a power-down assistance setup routine, the power-down assistance setup routine providing for one or more of:
sequence of steps a user of the MRI device of the plurality of MRI devices needs to perform to power-down the magnet, establishing a remote control by a remote user of the MRI device of the plurality of MRI device, or establishing an emergency communication session between the remote user and the user of the MRI device.
